(19) Europäisches Patentamt
European Patent Office
Office européen des brevets

(11) Publication number: **0 574 781 A2**

(12) **EUROPEAN PATENT APPLICATION**

(21) Application number: **93109079.9**

(51) Int. Cl.[5]: **C07D 403/04**, A61K 31/55

(22) Date of filing: **05.06.93**

(30) Priority: **16.06.92 US 899190**

(43) Date of publication of application: **22.12.93 Bulletin 93/51**

(84) Designated Contracting States: **AT BE CH DE DK ES FR GB GR IE IT LI LU MC NL PT SE**

(71) Applicant: **F. HOFFMANN-LA ROCHE AG**
**Postfach 3255**
**CH-4002 Basel(CH)**

(72) Inventor: **Hsu, Ming-Chu**
**445 East 86th Street, Apt. 15G**
**New York, N.Y. 10028(US)**
Inventor: **Huryn, Donna Mary**
**8 Tyler Court**
**Allentown, N.J. 08501(US)**
Inventor: **Tam, Steve Yik-Kai**
**13 Evergreen Road**
**West Caldwell, N.J. 07006(US)**

(74) Representative: **Mahé, Jean et al**
**Postfach 3255**
**Grenzacherstrasse 124**
**CH-4002 Basel (CH)**

(54) **2-Amino-5-heterocyclic-substituted-1,4-benzodiazepines and their use in the treatment of aids.**

(57) The benzodiazepines of formula

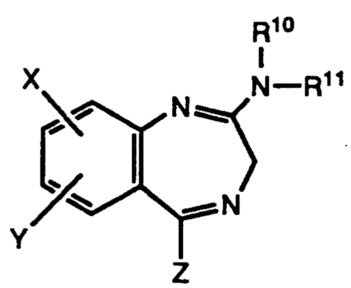

I

where
X and Y are each independently selected from H, halogen, and lower alkyl; $R^{10}$ and $R^{11}$ are both H, or one of $R^{10}$ and $R^{11}$ is H and the other is methyl, or $R^{10}$ and $R^{11}$ together are $-(CH_2)_n-$ where n = 4 or 5;
Z is a ring selected from the group consisting of

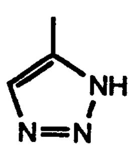

$(Z^1)$

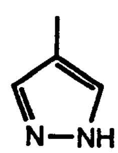

$(Z^2)$

or

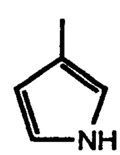

$(Z^3)$

where one or more carbon atoms in said ring Z can be substituted by F, Cl or lower alkyl;
as well as the pharmaceutically acceptable acid addition salts, carbamates, ureas and amides thereof can be

EP 0 574 781 A2

used as therapeutically active agent, especially as antiviral agents, particularly for the treatment, therapy or prophylaxis of AIDS and AIDS-related diseases.

The present invention relates to novel compounds of formula

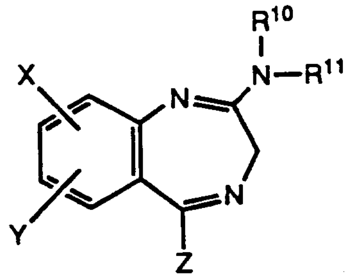

I

where

X and Y are each independently selected from H, halogen, and lower alkyl; $R^{10}$ and $R^{11}$ are both H, or one of $R^{10}$ and $R^{11}$ is H and the other is methyl, or $R^{10}$ and $R^{11}$ together are $-(CH_2)_n-$ where n = 4 or 5;

Z is a ring selected from the group consisting of

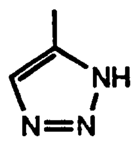

($Z^1$)

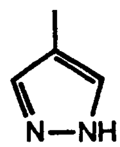

($Z^2$)

or

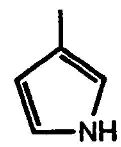

($Z^3$)

where one or more carbon atoms in said ring Z can be substituted by F, Cl or lower alkyl;

as well as the pharmaceutically acceptable acid addition salts, carbamates, ureas and amides thereof.

Objects of the present invention are the above compounds per se and for use as a therapeutically active agent, especially for the treatment or prophylaxis of viral infections, particularly of retroviral infections, such as HIV 1 and/or HIV 2 infections, or for protecting cells against such infections;

further a process for the manufacture of these compounds and medicaments containing one of such compounds and, optionally, a second antiviral agent, especially a reverse transcriptase inhibitor, such as ddC, AZT or ddl, TIBO derivatives, tricyclic diazepinones, a HIV-protease inhibitor, $\alpha$-, $\beta$- ad/or $\gamma$-interferon, interleukin-2 and/or GM-CSF, and

the use of these compounds for the manufacture of medicaments especially for the treatment or prophylaxis of viral infections, particularly of retroviral infections, such as HIV 1 and/or HIV 2 infections, or for protecting cells against such infections.

All the tautomeric and stereoisomeric forms of the compounds of formula I are included in the scope of this invention.

Pharmaceutically acceptable acid addition salts may be salts of pharmaceutically acceptable mineral acids such as hydrochloric and sulfuric acids. Pharmaceutically acceptable amides may be formed by neutralizing the compounds of the invention with organic acids such as lactic, acetic, malic or p-toluenesulfonic acid. Pharmaceutically acceptable carbamates may be formed by reacting the compounds of formula I with the product of a stoichiometric reaction between phosgene and an aliphatic or aromatic alcohol. Pharmaceutically acceptable ureas may be formed by reacting compounds of the invention with the product of a stoichiometric reaction between phosgene and an aliphatic or aromatic amine. The carbamtes, ureas and amides of the compounds of formula I are useful as pro-drugs.

As used in this specification, "lower alkyl" refers to a radical derived from a straight or branched carbon chain having from 1 to 7, preferably 1 to 4 carbon atoms including e.g. methyl, ethyl, propyl and isopropyl. "Acyl" means an organic radical derived from an organic acid by removal of a hydroxy group. Preferable organic acids include aliphatic and aromatic acid, for example acetic acid, sulfonacetic acid and benzoic acid.

Preferred compounds have the formula I, wherein Y is H and X is halogen or lower alkyl, particularly in the 7-position.

Particularly preferred compounds of formula I are those wherein $R^{10}$ is methyl, $R^{11}$ is hydrogen, ad X is selected from the group consisting of chlorine and methyl, particularly in the 7-position.

The especially preferred compounds of the invention have the formulae I(a), I(b) ad I(c):

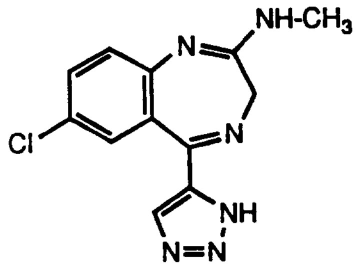

I(a)

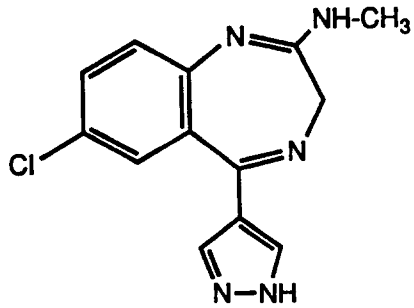

I(b)

and

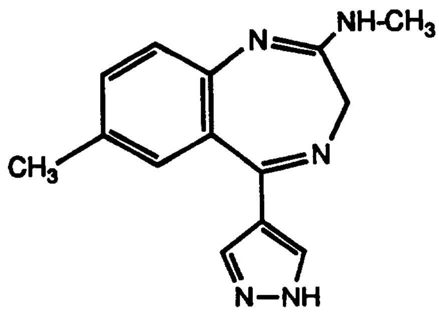

I(c)

The compounds of formula I may be prepared by reacting a 3H-1,4-benzodiazepin-2-(1H)-one of formula:

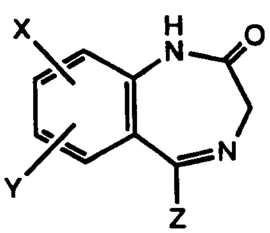

II

successively with $P_2S_5$ ad $HN(R^{10},R^{11})$ by conventional means, or by conversion of an amide of formula II following a variety of procedures known in the art. For example, a compound of formula II can be stirred with an amine of formula $HN(R^{10},R^{11})$ in a appropriate reaction-inert solvent in the presence of a suitable Lewis acid for example as described in U.S. Patent No. 3,644,335. Examples of appropriate reaction-inert solvents include, but are not limited to, tetrahydrofuran, toluene and dioxane. Examples of suitable Lewis acids include, but are not limited to, titanium tetrachloride, stannic chloride and the like.

The compounds of formula II can be prepared in a manner known per se, e.g. as described in US 3405122, 3398159, 3407211 and 3400128; in J. Org. Chem. 41, 1976, 2720; 35, 1970, 2455 and 46, 1981, 839; in Acta Chem. Scan. B 31, 1977, 701; in J. Hetereocyclic Chem. 12, 1975, 49 ad 25, 1988, 1293; in Synthesis 1988, 767; in Syn. Commun. 15, 1985, 1271 and J.A.C.S. 100, 1978, 4842, and as described in detail in Examples 1 to 3 below.

Thus the compounds of formula II can be prepared by cyclizing a compound such as that of formula 6 in Example 1, by acid-catalysis. This cyclization can be performed by heating the compound 6 with an acid, such as pivalic acid, in a solvent, such as toluene and THF, or in n-butanol, at a temperature up to reflux temperature. The amines, such as that of formula 6, can be prepared via the corresponding bromide, such as that of formula 5, 10, 10" or 17 below, starting from ketone of formula 4, 9 or 15.

The compounds of the invention have useful antiviral, especially anti-retroviral activity, particularly against HIV, the virus implicated in the development of AIDS and relates diseases such as ARC (AIDS relates complex). These compounds also inhibit HIV replication by inhibiting such important HIV viral functions as TAT (transactivating transcriptional) activity. The production of TAT by an infected cell

significantly activates the replication of HIV in a infected patient. By inhibiting TAT activity, the compounds of the invention dramatically diminish the replication of HIV and should thereby alleviate the destructive effects of HIV in a human patient.

The compounds of formula I were tested for anti-HIV-TAT activity in an assay described in US Patent No. 5070010, comprising the following steps:

(a) putting both the expression of the Secreted Alkaline Phosphatase (SeAP) gene and the viral trasactivator TAT gene under the control of the HIV promoter LTR responsive to the action of the HIV transactivator TAT;

(b) transfecting cultured mammalian cells with plasmids which contain the gene constructs of (a) above and cause cellular production of the trasactivating factor TAT ad SeAP;

(c) adding the agent to be tested, here the compounds of formula I; and determining the amount of SeAP produced, by measuring SeAP enzymatic activity, whereby inhibition of SeAP production correlates with the anti-TAT inhibition activity.

In this assay, the inhibition of SeAP positively correlates with anti-TAT activity. The greater the ability of a agent to inhibit SeAP, the greater is its anti-TAT activity.

Specifically, with respect to the results reported below, the anti-HIV-TAT assay was run as follows:

At 24 hours post transfection 1, 10 and 50 $\mu$M of a test compound of formula I was added to the culture media of COS cells transfected with two plasmids, one containing the reporter gene which codes for SeAP under control of HIV-LTR, and the other containing the HIV-TAT gene also under control of HIV-LTR. The alkaline phosphatase activity of the media was assayed 48 hours after addition of test compound with a colorimetric assay using p-nitrophenylphosphate as the substrate. The anti-TAT activity is measured by the percent inhibition of SeAP gene expression under the control of HIV-LTR versus the percent inhibition of SeAP gene under RSV-LTR, which does not respond to TAT.

The results in the Table below show that the compounds of formula I are specific inhibitors of HIV-TAT-regulated gene expression without non-specific cytotoxic effects.

The specificity of the compounds of formula I as TAT inhibitors was demonstrated with a parallel assay in which the SeAP gene expression is put under control of the Rous sarcoma virus (RSV)-LTR which does not respond to TAT. This assay thus eliminates the possibility that the compounds of formula I are either general cytotoxic agents or inhibit the activity of SeAP.

The anti-HIV-TAT activities of the test compounds were determined by measuring the amount of alkaline phosphatase in the supernatant media of cultures of cells in which SeAP gene expression was under the control of the HIV LTR promoter. The specific inhibitory activities of the test compounds were calculated according to the formula:

$$100 [(1-A/B) - (1-C/D)]$$

where A and B are the alkaline phosphatase activites produced by HIV-LTR/SeAP in the presence and absence, respectively, of test compound, and C and D are the alkaline phosphatase activities produced by RSV-LTR/SeAP in the presence and absence, respectively, of test compound. The concentrations tested ranged from 1 to 50 $\mu$M. The results provided are the average of at least three tests. In this table the designations "high" and "medium" indicate activity as follows: High is >60% inhibition; medium is 60-40% inhibition; low would be 40-20% inhibition. The test compound was added 24 hours after cells were transfected with the plasmids when SeAP specific mRNA and protein were already present and the protein was very stable. Therefore, 100% inhibition would not be observed with this assay procedure.

Table

| **Product of Example No.** | **Anti-HIV-TAT activity** |
|---|---|
| 1 | High |
| 2 | High/Medium |
| 3 | High/Medium |

Since the compounds of the invention effectively inhibit the TAT protein, so that viruses being affected cannot replicate themselves in the host cells, the compounds would be effective in the treatment of AIDS. In fact, compounds of the invention have been found to protect CD4+ lymphocytes in culture from the cytopathic effects of HIV. Moreover, since proteins closely related to TAT are found in other retroviruses,

such as HTLV-I, the compounds of the invention would likely inhibit the activity of these TAT-related proteins and, thus, be useful for treating these other retroviral infections, as well. The present invention also provides a method of alleviating the destructive effects of retroviral disease including HIV in a human patient, comprising treating the patient with an amount of a compound of the invention or a biologically active metabolite thereof sufficient to inhibit progression of the retroviral infection. This invention includes treatment or therapy of patients infected with HIV, including AIDS or ARC patients and patients with symptomatic or asymptomatic HIV infections. The compounds of this invention may be administered parenterally or orally in one or more doses at various intervals daily, preferably orally once daily. It is understood that in patients with liver or kidney problems, dosing and forms of administration may have to be adjusted to accommodate those conditions. With respect to a human patient, an antivirally effective amount of a compound of the invention that is administered e.g. orally is in the range of from about 0.5 to about 40 mg/kg body weight per day, preferably from about 1-15 mg/kg, particularly about 4-10 mg/kg, body weight per day. In unit dosage form, e.g. oral unit dosage form, for a 70 kg patient, this would be a amount of from about 35 to about 2,800 mg per day, preferably from about 210 to about 350 mg per day.

In addition, because the use of more than one active agent may provide a better therapeutic composition, and this is particularly true when the different agents act by different mechanisms, the present invention also includes antiviral compositions comprising both a compound according to the present invention together with one more other antiviral agents, such as ddC, AZT, 2',3'-dideoxyinosine (ddI), tetrahydroimidazobenzodiazepine-one (TIBO) derivatives, HIV-protease inhibitors and tricyclic diazepinones, HIV-integrase and RNase H inhibitors, as well as biological response modifiers, including, for example, alpha-, beta- or gamma-interferon, interleukin-2 and granulocyte-macrophage colony stimulating factor (GM-CSF) and the like.

With respect to human patients, the compounds of this invention may be administered in conventional dosage forms, such as those set forth herein. Either the compounds, compositions, or their pharmaceutically acceptable acid addition salts, carbamates, ureas or amides are suitable.

The compounds of the invention are administered in the dosages as set forth herein until improvement of patient condition and/or alleviation of viremia. The compounds may also be administered with other antiviral and/or biological response modifiers as noted above. The dosages of ddC ad AZT used in AIDS or ARC human patients have been published. When given in combined therapy, the other anti-HIV compounds may be given at the same time as a compound of the invention or the dosing may be staggered as desired. The two (or more) drugs may also be combined in a composition. Doses of each drug may be less when used in combination than when they are used as a single agent.

The instant invention is also directed to compositions containing a therapeutically effective amount of a compound of the invention in a pharmaceutically acceptable carrier. It is possible for the compounds of the invention to be administered alone in solution. However, it is preferred that the active ingredients be administered in a pharmaceutical formulation. In the context of the instant invention, formulation means composition. These formulations comprise at least one active ingredient of the invention together with one or more pharmaceutically acceptable carriers and excipients and may optionally include other therapeutic agents, for example an HIV-RT or HIV-protease inhibitor. As included within the scope of this invention, "acceptable" is defined as being compatible with other ingredients of the formulation and not injurious to the organism or host cell being treated. These carriers include those well known to practitioners in the art as suitable for oral, rectal, nasal, topical, buccal, sublingual, vaginal, or parenteral (including subcutaneous, intramuscular, intravenous, ad intradermal) administration. The compositions may be conveniently presented in unit dosage form and prepared by methods known in the pharmaceutical art. Such methods include the preparation of the active ingredient in a carrier which may contain additional medicinally active ingredients, for example, ddC, AZT, ddI, interferon, IL-2 or a HIV-protease inhibitor.

Examples of compositions of the invention are solutions of the active ingredient(s), e.g. in water or saline; capsules, e.g. soft gelatine capsules; sachets or tablets, each containing a pre-determined amount of the active ingredient, e.g. as granules; solutions or suspensions in an aqueous liquid or in an oil-in-water emulsion or a water-in-oil liquid emulsion. Tablets may include one or more of lactose, microcrystalline cellulose, colloidal silicon dioxide, croscarmellose sodium, magnesium stearate, stearic acid ad other excipients, colorants ad pharmacologically compatible carriers. Formulations suitable for oral administration include lozenges comprising the active ingredient in a flavor, usually sucrose and acacia or tragacanth; pastilles comprising the active ingredient in an inert basis such as gelatin and glycerin, or sucrose and acacia; and mouthwashes comprising the active ingredient in a suitable liquid carrier. Formulations for rectal administration may be presented as a suppository with a suitable base comprising cocoa butter or a salicylate. Formulations suitable for vaginal administration may be presented as pessaries, tampons, creams, gells, pastes, foams or spray formulas. Formulations suitable for parenteral administration include

aqueous and non-aqueous, isotonic sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose sealed containers, for example ampules and vials, and may be stored in a lyophilized condition requiring only the addition of the sterile liquid carrier, for example water for injections, immediately prior to use. Extemporaneous injection solutions and suspensions may be prepared from sterile powder, granules and tablets of the kind previously described.

Example 1

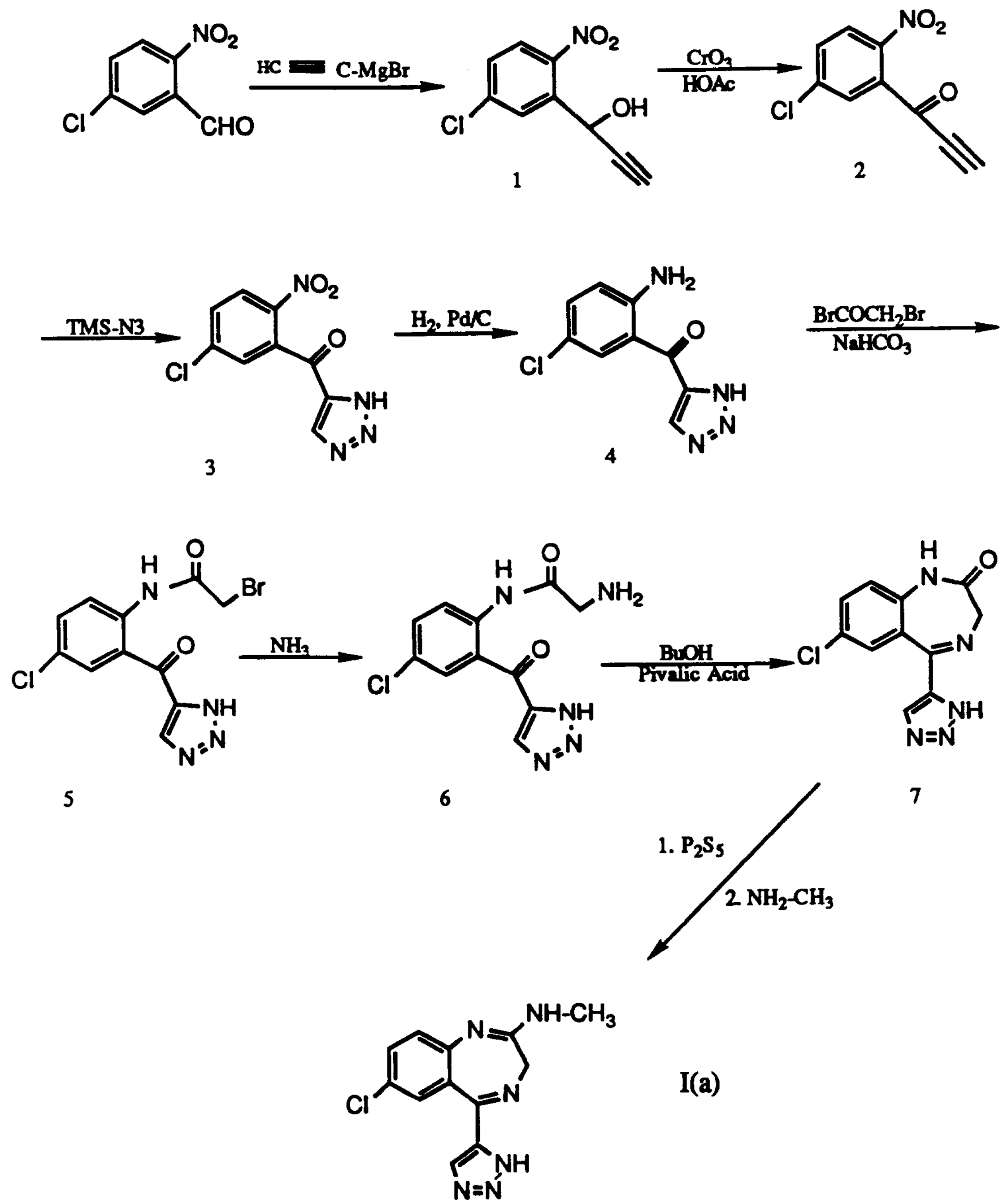


1 2 3 4 5 6 7 I(a)

a) Acetylene, was bubbled into 75 ml of THF. EtMgBr (50 ml) was added to the THF/acetylene mixture over a period of 1 hour such that the temperature of the reaction mixture never exceeded 40 °C. During this addition acetylene was continually bubbled through the mixture. The mixture was allowed to stir at

room temperature for 30 additional minutes after which the flow of acetylene was stopped, and the mixture cooled to 0°C. A solution of 5-chloro-2-nitrobenz-aldehyde (13.2g) in 70 ml THF was added dropwise to the mixture. The reaction was allowed to warm to room temperature overnight, then quenched with saturated $NH_4Cl$ solution and extracted with EtOAc. After drying, filtration and evaporation, the residue was purified via flash column chromatography (10% EtOAc/Hexane), to provide 12.26 g of 5-chloro-alpha-ethynyl-2-nitrobenzenemethanol, IR(KBr) 3290, 2120, 1525, 1338 $cm^{-1}$.

b) A solution of 5-chloro-alpha-ethynyl-2-nitrobenzene-methanol (1.54g) in 60 ml glacial acetic acid was heated to 40°C, and then treated with 1.44g $CrO_3$. After stirring, the solution was cooled to room temperature, then extracted with $CH_2Cl_2$. The organic fraction was extracted with $H_2O$ ad saturated $NaHCO_3$, then dried, filtered and evaporated. Column chromatography using a gradient elution system (9% EtOAc/Hexane, 17% EtOAc/Hexane) provided 0.94 g of 1-(5-chloro-2-nitrophenyl)-2-propyn-1-one, MS calc. 208.9880; found 208.9872.

c) 1-(5-Chloro-2-nitrophenyl)-2-propyn-1-one (0.03 g) was mixed with azido-trimethylsilane (0.02 ml) and $CH_3CN$ (5 ml), and the mixture was heated to 100°C under a stream of argon for three hours. The solution was then evaporated, and subsequently purified via flash column chromatography using a gradient elution system (5% EtOAc/Hexane, 20% EtOAc/Hexane, 50% EtOAc/Hexane) to provide 0.03g of (5-chloro-2-nitrophenyl)-(1H-1,2,3-triazol-5-yl)-methanone, MS 252($M^+$).

d) A mixture of the (2-nitro-5-chlorophenyl)-(1H-1,2,3-triazol-5-yl)-methanone (2.53 g), EtOH (100 ml) ad 10% Pd on C (50 mg) was hydrogenated under 40 psi hydrogen for 16 hours. The solution was then filtered, evaporated and slurried with $CH_2Cl_2$. The resultant solids were collected yielding 1.64 g of (2-amino-5-chlorophenyl)-(1H-1,2,3-triazol-5-yl)-methanone. The mother liquors were purified by column chromatography using a gradient elution system (9% EtOAc/Hexane, 20% EtOAc/Hexane, 33% EtOAc/Hexane) to provide another 0.48 g of product, mp 148-150°C;

e) Subsequently, to a solution of 1.0 g of (2-amino-5-chlorophenyl)-(1H-1,2,3-triazol-5-yl)methanone , in 150 ml of $CH_2Cl_2$ ad 60 ml of THF, was added 50 ml of ice water and 2.0 g of sodium bicarbonate. To the stirred two-phase mixture was added 0.98 ml of bromoacetyl bromide. The reaction mixture was stirred for 0.5 hours. The layers were separated, and the aqueous portion was extracted with $CH_2Cl_2$. The organic extracts were washed with water, followed by brine, then dried and evaporated. The residue was purified by filtration using ethyl acetate-hexane as eluant to provide 1.2 g of 2-bromo-4'-chloro-2'-[-(1H-1,2,3-triazol-5-yl)carbonyl]acetanilide , mp = 173-175°C.

f) To 100 ml of condensed liquid ammonia in a dry-ice bath was added a solution of 1.2 g of 2-bromo-4'-chloro-2'-[(1H-1,2,3-triazol-5-yl)carbonyl]acetanilide in 25 ml of THF. The reaction mixture was stirred overnight and the ammonia allowed to evaporate. The remaining THF was evaporated ad the combined residues were stirred with 100 ml of 10% methanol-ethyl acetate and 20 ml of water. The aqueous portion was extracted with 10% methanol-ethyl acetate. The organic extracts were washed with brine, dried and evaporated. The residue was heated to reflux in 60 ml n-butanol and 100 mg of pivalic acid for 16 hours. The butanol was evaporated and the residue was pumped dry under high vacuum. Fractional crystallization of the residue from 8% methanol-dichloromethane gave 705 mg of 7-chloro-5-(1H-1,2,3-triazol-5-yl)-1,3-dihydro-2H-1,4-benzo-diazepin-2-one, mp 246-249°C after charcoal treatment and recrystallization from methanol.

g) A mixture of 7-chloro-1,3-dihydro-5-(1H-1,2,3-triazol-5-yl)-2H-1,4-benzodiazepin-2-one (200 mg), $P_2S_5$ (222 mg) and THF (25 ml) was sonicated at 20-40°C for 2 h. The solvent was evaporated, and the residue was stirred in a mixture of EtOAc (200 ml) and $H_2O$ (100 ml). The undissolved yellow solids were collected and dried. This material was dissolved in THF (15 ml), then added to a -70°C solution of THF (20 ml) into which $NH_2CH_3$ had been bubbled through for 10 min. The solution was stirred at -70°C, then allowed to warm to room temperature. The solids which precipitated were collected and combined with $H_2O$ ad heated on a steam bath. After cooling, the solids were collected and recrystallized from methanol to give 89 mg of 7-chloro-N-methyl-5-(1H-1,2,3-triazol-5-yl)3H-1,4-benzodiazepin-2-amine, mp = 232-235°C.

Example 2

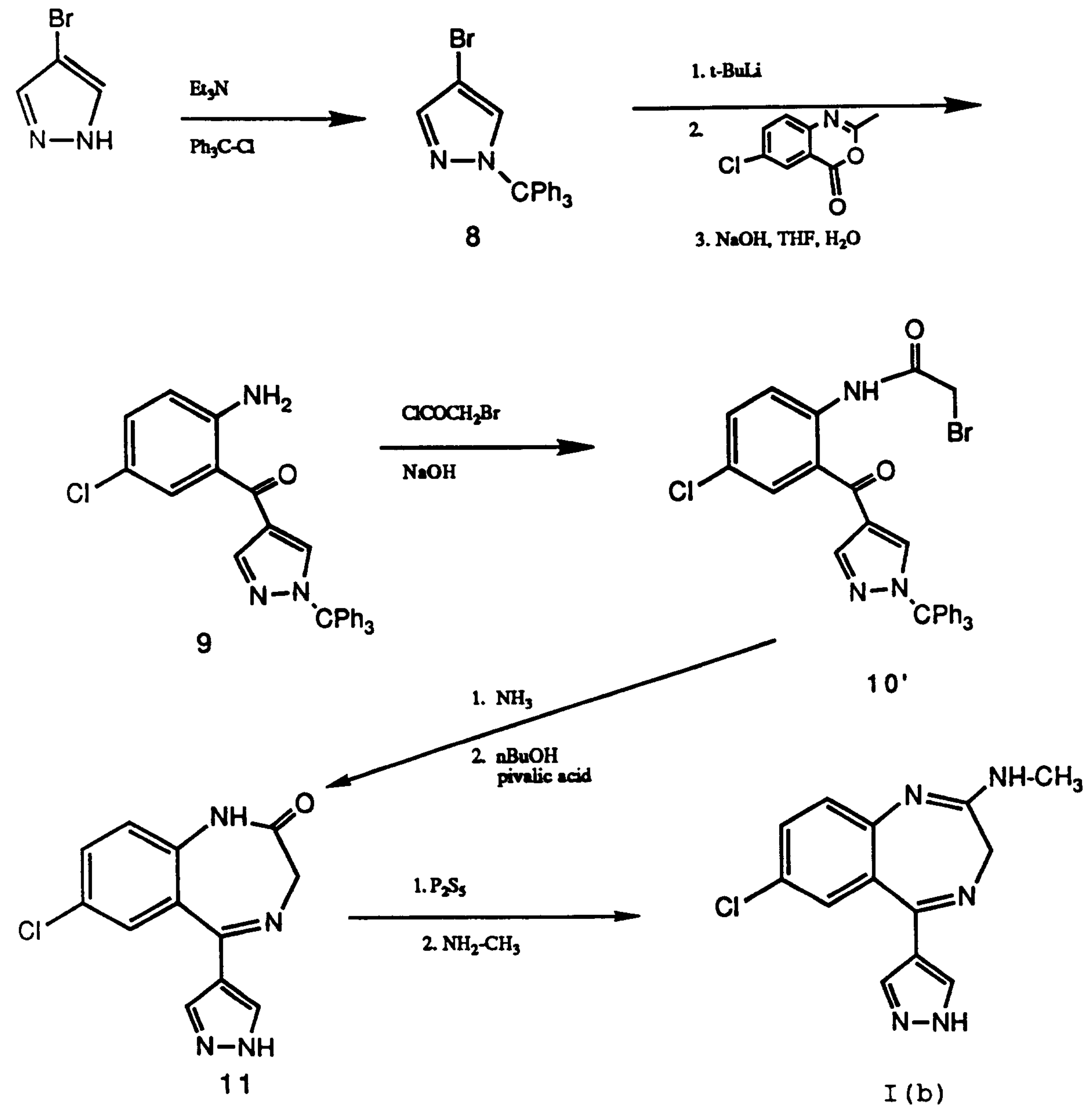


a) A mixture of 4-bromo(1H)-pyrazole (50.8g), 1300ml of $CH_2Cl_2$, triphenylmethyl chloride (99.5g) and $Et_3N$ (35.3g) was stirred at room temperature for 24 hours. The solution was then extracted with $H_2O$, dried, filtered and evaporated. Crystallization of the residue with $CH_2Cl_2$/Hexane provided white crystals: mp190-192°C. A second crop was taken to provide a total of 117.3g of 4-bromo-1-(triphenylmethyl)-1H-pyrazole.

b) A mixture of 4-bromo-1-(triphenylmethyl)-1H-pyrazole (48g), $Et_2O$ (100ml) ad THF (500ml) was stirred while cooling to -78°C under a stream of argon. tBuLi (160ml) was added to the mixture, and the resultant red solution was stirred for 2.5 hours at -78°C. At that time, the solution was added to a solution of 2-methyl-6-chloro-4H-3,1-benzoxazin-4-one (21g) in THF (500ml) which had been cooled to -78°C. The mixture was allowed to warm to room temperature overnight, and then quenched with saturated $NH_4Cl$ solution. After dilution with EtOAc, the layers were separated, and the organic layer washed with saturated NaCl solution, dried, filtered and evaporated. The obtained solid was combined with THF (400ml), MeOH(350ml), $H_2O$(250ml) and 10n NaOH (300ml), and stirred at reflux temperature for 3 hours. After cooling to room temperature, the organic and aqueous phases were separated. The

aqueous phase was extracted with $Et_2O$ and the organic fractions dried, filtered and evaporated. The obtained foam was combined with $CH_2Cl_2$ and stirred overnight. After filtration, the filtrate was evaporated to afford 49 g of compound [9] as an oil.

c) To a stirred mixture of compound [9], THF (450 ml), $CH_2Cl_2$ (450ml), ad 1N NaOH (1400 ml) was added dropwise, 10.5ml of bromoacetyl chloride all at room temperature. The two-phase mixture was stirred at room temperature for 20 minutes. After separation of the layers, the aqueous layer was extracted with $CH_2Cl_2$. The organic layers were dried ad evaporated to dryness. The residue was crystallized from THF and hexane to afford 23.5 g of 2-bromo-4'-chloro-2'-[(1-(triphenylmethyl)-1H-pyrazol-4-yl]carbonyl]acetanilide, mp 197-200 °C.

d) To 1 liter of liquid ammonia in a dry-ice bath was added a solution of 2-bromo-4'-chloro-2'-[(1-(triphenylmethyl)-1H-pyrazol-4-yl]carbonyl]acetanilide (23.5 g) in THF (200ml). The reaction mixture was stirred overnight and the ammonia allowed to evaporate. Residual solvent was distilled off. The residue was stirred with EtOAc and $H_2O$. The product was washed with water and dried to give 18.3 g of a solid. A suspension of this material in 1-butanol (600ml) containing 300mg of pivalic acid was heated to reflux temperature for 8 hours. Additional portions of 300mg each of pivalic acid were added after 3 hours and 5 hours. Volatiles were evaporated, and trituration of the residue yielded 6.5 g of product. This was dissolved in MeOH, treated with charcoal, filtered and concentrated. The precipitated product was collected to give 5.25 g of 7-chloro-1,3-dihydro-5-(1H-pyrazol-4-yl)-2H-1,4-benzodiazepin-2-one, mp 289-291 ° (d).

e) A mixture of 7-chloro-1,3-dihydro-5-(1H-pyrazol-4-yl)-2H-1,4-benzodiazepin-2-one (295 mg), $P_2S_5$ -(327mg) and THF (25 ml) was sonicated at 20-40 °C for two hours. The solvent was evaporated, and the residue stirred in a mixture of EtOAc (200 ml) ad $H_2O$ (100 ml). The undissolved solids were collected ad dried. This material was dissolved in THF (15 ml), then added to a -70 °C solution of THF (25 ml) to which $NH_2CH_3$ had been bubbled through for 10 min. The solution was stirred at -70 °C for 15 minutes, then allowed to warm. After evaporation of the solvent, the residue was combined with $H_2O$ and heated on a steam bath. The precipitated solids were collected, dissolved in EtOAc, dried and evaporated. The residue was purified by recrystallization from MeOH-$CH_3CN$ to provide 7-chloro-N-methyl-5-(1H-pyrazol-4-yl)-3H-1,4-benzodiazepin-2-amine (38 mg), mp 287 - 289 °C.

The benzodiazepinone intermediate of paragraph d) above was also prepared as follows:

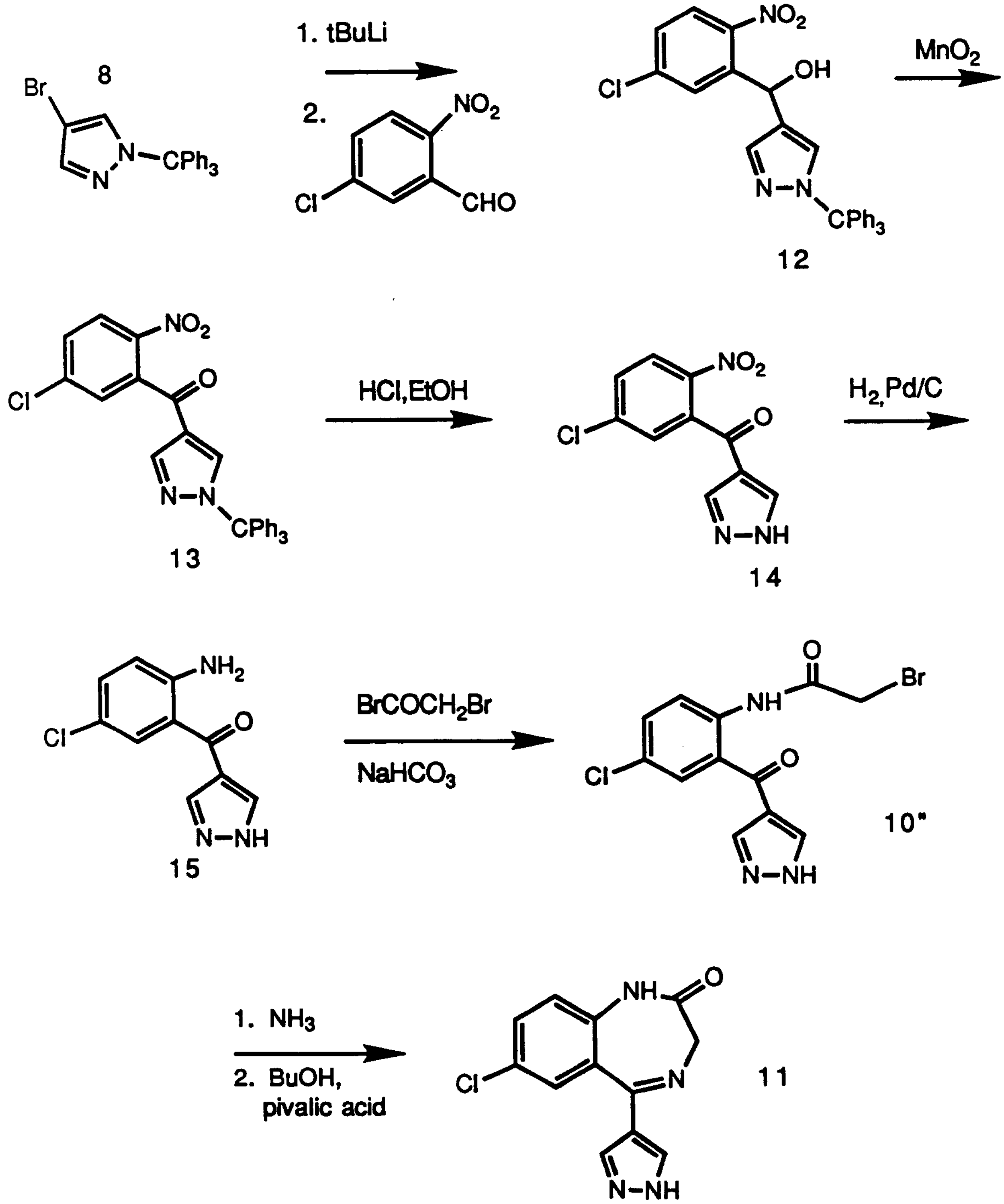


8 12 13 14 15 10" 11

a) Compound [8] (24g) was combined with THF (400 ml) and $Et_2O$ (100 ml) and cooled to -78 °C. tBuLi (80 ml) was added dropwise to the mixture, and the resultant red solution stirred for 2.5 hours at -78 °C. A solution of 5-chloro-2-nitrobenzaldehyde (11.2 g) in THF (150 ml) was added dropwise to the solution, and the resultant mixture allowed to warm to room temperature overnight. After quenching with saturated $NH_4Cl$ solution, the mixture was diluted with EtOAc, and the layers separated. The organic fraction was washed with saturated NaCl solution, dried, filtered and evaporated. Flash column chromatography using a gradient elution system from 10% EtOAc/Hexane to 75% EtOAc/Hexane provided 20.6g of alpha-(5-chloro-2-nitrophenyl)-1-(triphenylmethyl)-1H-pyrazole-4-methanol as a white foam. MS 495($M^+$).

b) A mixture of the product of a) (27.1g), $CHCl_3$ (250ml) and $MnO_2$ (20g) was stirred at reflux temperature for three hours. An additional aliquot of $MnO_2$ (5g) was added, and the mixture stirred an additional five hours. After cooling, the mixture was filtered and evaporated to provide 26.9g (5-chloro-2-nitrophenyl) [1-(triphenylmethyl)-1H-pyrazole-4-yl]methanone, MS 493($M^+$).

c) A mixture of the product of b) (26.9g), EtOH (400ml) and $NH_4Cl$ (100ml) was combined ad stirred at reflux temperature for three hours. After neutralization with 30% NaOH (50ml) at 0°, the EtOH was evaporated and the resultant aqueous phase extracted with EtOAc. The organic fractions were dried, filtered and evaporated. The residue was purified by filtration through silica gel using a gradient elution system from 30% EtOAc/Hexane to 100% EtOAc to yield (5-chloro-2-nitrophenyl)(1H-pyrazol-4-yl)-methanone. This was combined with EtOH (250ml) ad 10% Pd on C (100 mg) and hydrogenated at 45psi. Flash column chromatography afforded 10g of (2-amino-5-chlorophenyl)-1H-pyrazol-4-yl-methanone, MS 221 ($M^+$).

d) To a solution of the product of c) (10.5g) in THF (300 ml) and $CH_2Cl_2$ (300ml) was added $NaHCO_3$ - (25g) ad a ice-water mixture (300ml). The stirred two phase mixture was treated with 37.2ml $BrCOCH_2Br$. The two phases were separated, and the aqueous phase extracted with $CH_2Cl_2$. The organic fractions were dried, filtered and evaporated. The residue was dissolved in THF (100ml) and added to liquid $NH_3$ - (200ml), which had been cooled to -78°C, and allowed to stir overnight while warming to room temperature. The volatiles were evaporated and the residue partitioned between EtOAc and $H_2O$. After separation of the layers, the aqueous fraction was extracted with EtOAc. The organic fractions were dried, filtered and evaporated. The residue was combined with 1-BuOH (100ml) ad pivalic acid (75mg) and the mixture heated to reflux temperature. The solvent was removed by evaporation, ad the product purified by flash column chromatography (6.5% $MeOH/CH_2Cl_2$) to provide 5.5g of the desired benzodiazepinone.

Example 3

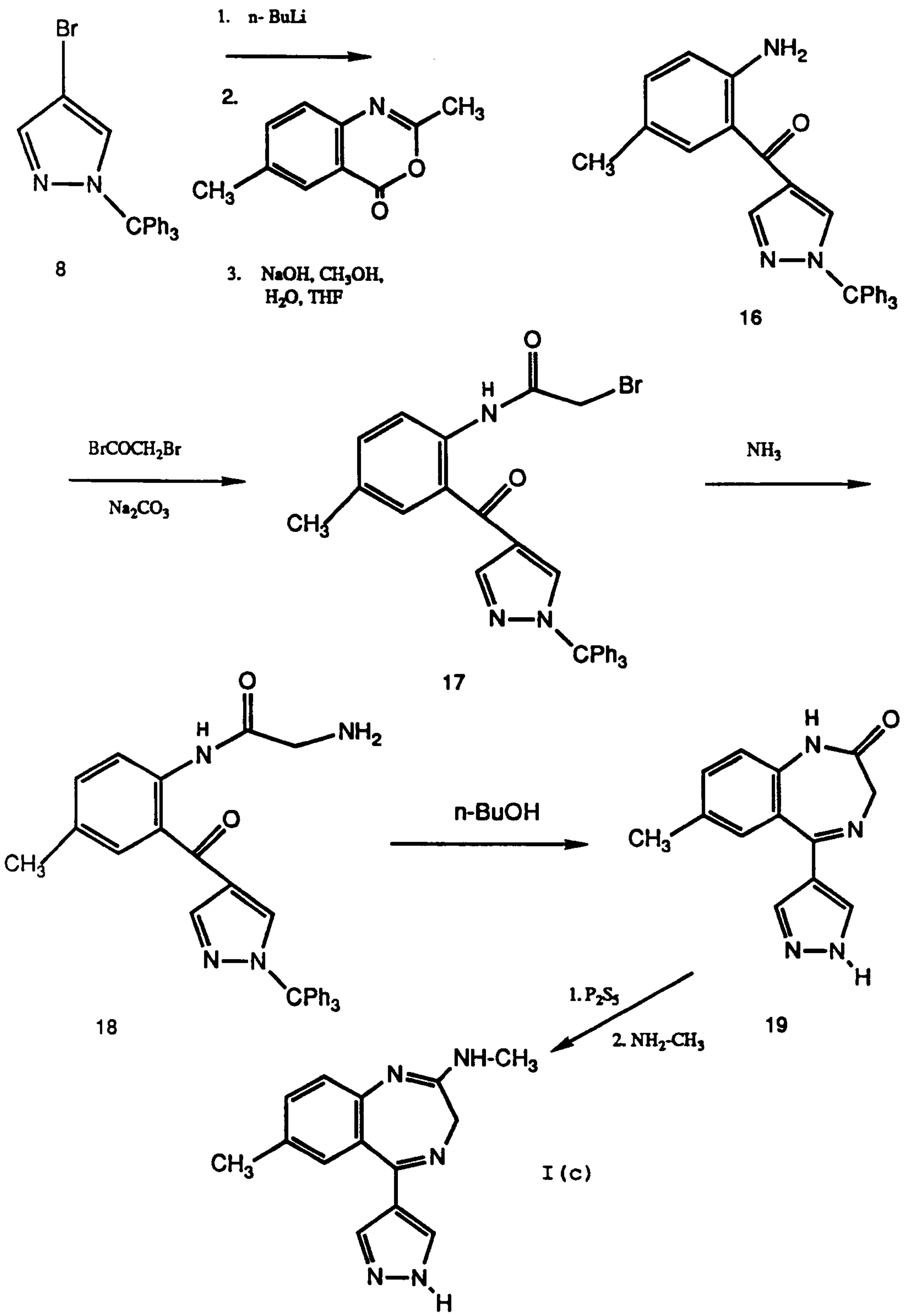


8 16 17 18 19 I(c)

a) Bromopyrazole (24.0g) was suspended in dry THF (600ml) and cooled in dry ice-acetone bath with stirring under an argon atmosphere. n-Butyllithium (2.5M in hexane) was added dropwise. After stirring for 2 hours with cooling in a dry ice-acetone bath, the THF solution was added to 2-methyl-6-methyl-4H-3,1-benzoxazin-4-one (8.76g) in THF (500ml), pre-cooled to about -50 °C over 10 minutes. The reaction was quenched after stirring for 20 minutes by addition of 15% ammonium chloride in water (w/v, 300ml)

and allowed to warm to room temperature. The reaction mixture was diluted with EtOAc and the layers were separated. The organic layer was washed with saturated aqueous sodium chloride. Aqueous layers were washed with EtOAc. The organic layers were combined, dried, filtered, and concentrated. The resulting solid was suspended in a mixture of THF (300ml), methanol (350ml), water (250ml) and 10N sodium hydroxide (270ml), ad heated at reflux temperature with stirring for 6 to 24 hours. The resulting mixture was allowed to cool to room temperature, and partitioned between ether and water. The organic layers were washed with saturated aqueous sodium chloride, then combined, dried, filtered, and concentrated. Residue was suspended in $CH_2Cl_2$ ad filtered. The filtercake was washed with $CH_2Cl_2$. The filtrate was concentrated ad passed through silica gel using EtOAc-$CH_2Cl_2$ mixture (1:9 v/v) as eluant. The eluant was combined and concentrated ad the resulting residue crystallized from $CH_2Cl_2$-hexane to give 15.18g (of 2-amino-5-methylphenyl)[1-(triphenylmethyl)-1H-pyrazol-4-yl]methanone, MS Calcd: 443. 1997; Found: 443.1990.

b) The product of a) (2.85g) was dissolved in a mixture of THF (150ml) and ether (150ml) and cooled in an ice-water bath. Saturated aqueous sodium carbonate (100ml) was then added. Bromoacetyl bromide (4 x 0.67ml) was added under stirring. After 4 hours, the reaction mixture was diluted with water and extracted with EtOAc. The precipitate formed was collected by filtration and dissolved in $CH_2Cl_2$. The EtOAc fraction was combined with the $CH_2Cl_2$ solution and dried, filtered, evaporated, and concentrated. The residue was crystallized from $CH_2Cl_2$-hexane to yield 3.33g of 2-bromo-N-[4-methyl-2-[[1-(triphenylmethyl)-1H-pyrazol-4-yl]carbonyl]phenyl]acetamide, MS Calcd: 563.1208; Found: 563.1188.

c) The product of b) (2.26g) was dissolved in dry $CH_2Cl_2$ (100ml) and cooled in a dry ice-acetone bath. Liquid ammonia (50 ml) was condensed into the reaction mixture. The resulting solution was stirred and allowed to warm to room temperature overnight. Water was added, ad the remaining precipitate dissolved. After mixing, the layers were separated. The organic layer was extracted with saturated aqueous sodium bicarbonate. The aqueous layers were washed with $CH_2Cl_2$. The $CH_2Cl_2$ layers were combined, dried, filtered, and concentrated. The residue was recrystallized from $CH_2Cl_2$-hexane to give 1.68 g of 2-amino-N-[4-methyl-2-[[1-(triphenylmethyl)-1H-pyrazol-4-yl]-carbonyl]phenyl]acetamide, MS Calcd: 501.2291; Found: 501.2272.

d) A suspension of the product of c) (15.02g) in nBuOH (300 ml) was heated at reflux with stirring for 64 hours. Alter cooling to room temperature, the reaction was concentrated to dryness. The residue was suspended in THF and heated to reflux. The resulting suspension was filtered. The filtercake was washed with THF. The filtrate was washed and heated at boiling temperature and concentrated. The resulting mixture was allowed to cool to room temperature and allowed to stand for 3 hours. The resulting product was washed with THF and dried yielding 5.50 g of 1,3-dihydro-7-methyl-5-(1H-pyrazol-4-yl)-2H,1,4-benzodiazepin-2-one, mp 282-289 °C.

e) Phosphorous pentasulfide (0.5 g) was added to a suspension of the product of d)(0.24 g) in THF (50 ml). The mixture was kept in an ultrasound bath for one hour, then concentrated to dryness. This residue was partitioned between THF-EtOAc (1:2, v/v, 150 ml) and half saturated $NaHCO_3$ solution. The organic layer was washed with sat. NaCl solution (100 ml). The aqueous layers were washed with the THF-EtOAc mixture and the organic layers were concentrated and kept in a vacuum desicator overnight. The residue was dissolved in THF and cooled to -40 °C. Methylamine gas was bubbled into the THF solution for 10 min. The gain in weight was 6 g. The mixture was allowed to warm up to room temperature ad stirred for two hours. After concentration the residue was dissolved in a mixture of THF-EtOAc (1:2 v/v, 100 ml) and extracted with sat. $NaHCO_3$ solution (100 ml) ad sat. NaCl solution (100 ml). The aqueous layers were washed with the THF-EtOAc mixture, and the organic layers dried, filtered, and concentrated. The residue was chromatographed on silica gel, using $CH_3OH$-$CHCl_3$ (v/v 1:9) as eluant to give, after recrystallization from $CH_3OH$-$CHCl_3$-hexane, N,7-Dimethyl-5-(1H-pyrazol-4-yl)-3H-1,4-benzodiazepin-2-amine (0.22 g), mp 262-270 °C.

The following galenical compositions containing a compound of the invention as active ingredients as defined above, can be prepared in a manner known per se:

a) Oral liquid formulation:

| Ingredients | mg/formulation |
|---|---|
| Active ingredient | 20.0 mg |
| Methylparaben | 20.0 mg |
| Sucrose | q.s. |
| Flavoring agent | q.s. |
| Citrate buffer | q.s. |
| Purified water q.s. | 5.0 ml |

b) Tablet formulation:

| Ingredients | mg/tablet |
|---|---|
| Active ingredient | 20 mg |
| Starch | 40 mg |
| Avicel | 80 mg |
| Lactose | 274 mg |
| Magnesium stearate | 2 mg |
| | 416 mg |

c) Soft gelatine capsule formulation:

| Ingredients | mg/capsule |
|---|---|
| Active ingredient | 20 mg |
| Ethoxylated Fatty acids | 500 mg |
| PEG 4000 | 100 mg |
| Vegetable oils q.s. to | 1.0 ml |

## Claims

**1.** A compound of formula

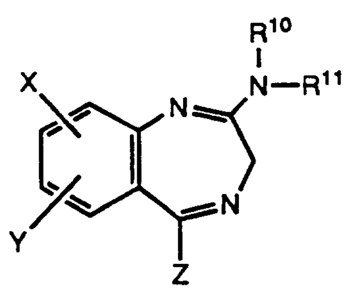

I

where

X and Y are each independently selected from H, halogen, and lower alkyl; $R^{10}$ and $R^{11}$ are both H, or one of $R^{10}$ and $R^{11}$ is H and the other is methyl, or $R^{10}$ and $R^{11}$ together are $-(CH_2)_n-$ where n = 4 or 5;

Z is a ring selected from the group consisting of

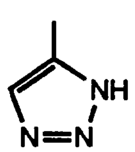

($Z^1$)

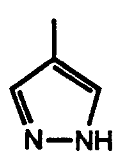

($Z^2$)

or

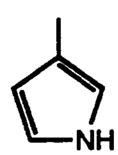

($Z^3$)

where one or more carbon atoms in said ring Z can be substituted by F, Cl or lower alkyl;
as well as the pharmaceutically acceptable acid addition salts, carbamates, ureas and amides thereof.

**2.** A compound according to claim 1 wherein Y is H and X is selected from the group consisting of halogen and lower alkyl, particularly wherein X is in the 7-position.

**3.** A compound according to claim 2 wherein $R^{10}$ is methyl, $R^{11}$ is hydrogen, and X is selected from the group consisting of chlorine and methyl, particularly in the 7-position.

**4.** A compound according to claim 3, having one of the formulae I(a), I(b) or I(c):

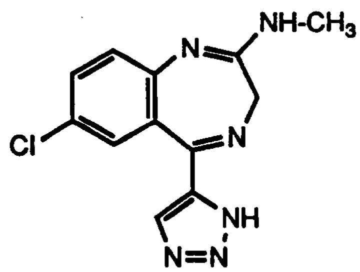

I(a)

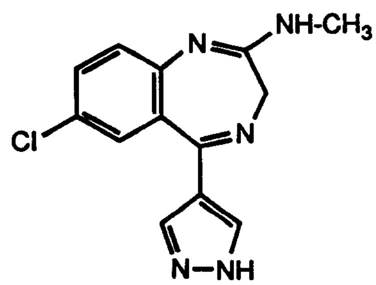

I(b)

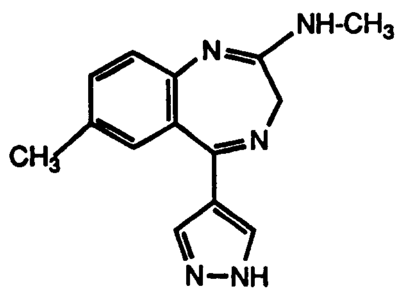

I(c)

**5.** A compound according to any one of claims 1 to 4 for use as a therapeutically active agent, especially as antiviral agents, particularly for the treatment, therapy or prophylaxis of AIDS and AIDS-related diseases.

**6.** A process for preparing a compound as in claim 1, which comprises reacting a compound of formula

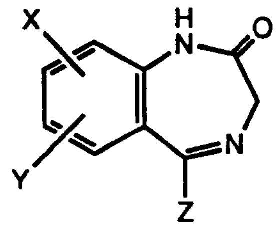

II

successively with $P_2S_5$ and an amine $HN(R^{10},R^{11})$, or by reacting a compound of formula II with an amine $HN(R^{10},R^{11})$ in a reaction-inert solvent in the presence of a Lewis acid, and where an amide of a compound of formula I is desired, neutralizing a compound of formula I with a organic acid, and where a carbamate or a urea is desired, reacting a compound of formula I with the product of the stoichiometric reaction between phosgene and an aliphatic or aromatic alcohol, or an aliphatic or aromatic amine, respectively.

**7.** A medicament, especially for the treatment or prophylaxis of viral infections, particularly of retroviral infections, such as HIV 1 and/or HIV 2 infections, or for protecting cells against such infections, or for alleviating the cytopathic destructive effects of retroviral disease in a patient infected with a retrovirus, containing as active pharmaceutical ingredient a compound as in claim 1 and, optionally, a second antiviral agent, especially a reverse transcriptase inhibitor, such as ddC, AZT, a HIV-protease inhibitor, $\alpha$-, $\beta$- and/or $\gamma$-interferon, interleukin-2 and/or GM-CSF.

**8.** The use of a compound as in claim 1 for the manufacture of a medicament, especially for the treatment or prophylaxis of viral infections, particularly of retroviral infections, such as HIV 1 and/or HIV 2 infections, or for protecting cells against such infections, or for alleviating the cytopathic destructive effects of retroviral disease in a patient infected with a retrovirus.